# EUROPEAN PATENT APPLICATION

(11) **EP 2 586 475 A1**
(43) Date of publication of application: **01.05.2013**
(21) Application number: 11187239.6
(22) Date of filing: 31.10.2011
(51) Int. Cl.: A61M 5/00

(54) **Storage device for single-use needle assemblies**

(71) Applicant: Sanofi-Aventis Deutschland GmbH, 65929 Frankfurt am Main (DE)
(72) Inventor: The designation of the inventor has not yet been filed

(57) **Abstract**

Described is a needle assembly storage device (2) comprising a case (2.1) having a first port (2.6) and a second port (2.7), a first spool (1.7.1) rotatably disposed in the case (2.1) and adapted to support a storage unit (1) of needle assemblies, a second spool (1.7.2) rotatably disposed in the case (2.1) and adapted to support the storage unit (1) of the needle assemblies, and an actuator (2.3) adapted to rotate the first spool (1.7.1) or the second spool (1.7.2) to advance the storage unit (1) past the first port (2.6) and the second port (2.7).

## Description

### Technical Field

The invention relates to a storage device for single-use needle assemblies.

### Background of the Invention

Patients suffering from diseases like diabetes have to frequently self-administer injections. Injection devices like auto-injectors or pen injectors have been developed to facilitate self-administering injections. Typically, such injection devices are fitted with a sterile, disposable needle assembly for each injection to minimize the risk of infections.

A conventional needle assembly consists of a hub for engaging the injection device, a double-tipped needle coupled to the hub, and a spring-biased needle shield for hiding the needle from view and covering an injection end of the needle after the injection. The conventional needle assembly also includes a protective cap to maintain sterility of the needle and prevent against inadvertent actuation. Conventional needle assemblies are often packaged loosely in boxes. Thus, a patient is required to carry a box of the needle assemblies (and a sharps disposal unit) when travelling. Similarly, loosely packing the needle assemblies does not optimally use packing space available. Thus, there is a need for a single-use needle assembly.

Further, a conventional needle assembly typically engages the injection device by threads, requiring the patient to manually engage and disengage the needle assembly and the injection device. However, it may present an injury risk if the patient is required to manually remove a single-use needle, and in any event, it may be difficult for the patient (especially if the patient has dexterity or vision problems) to manipulate the single-use needle. Thus, there is a need for a storage device for single-use needle assemblies.

### Summary of the Invention

It is an object of the present invention to provide a storage device for single-use needle assemblies.

In an exemplary embodiment, a needle assembly storage device according to the present invention comprises a case having a first port and a second port, a first spool rotatably disposed in the case and adapted to support a storage unit of needle assemblies, a second spool rotatably disposed in the case and adapted to support the storage unit of the needle assemblies, and an actuator adapted to rotate the first spool or the second spool to advance the storage unit past the first port and the second port.

In an exemplary embodiment, the needle assembly storage device further comprises a separator disposed in the case adjacent the first port. The separator is adapted to separate a container surrounding the needle assemblies. At least one roller is disposed in the case adjacent the second port, the at least one roller adapted to re-wrap the needle assemblies in the container.

In an exemplary embodiment, the needle assembly storage device further comprises a sleeve stopper disposed in the case adjacent the first port. The sleeve stopper is adapted to releasably engage a given one of the needle assemblies when the given one of the needle assemblies is aligned with the first port.

In an exemplary embodiment, the needle assembly storage device further comprises a needle stopper disposed in the case adjacent the first port, the needle stopper adapted to engage a given one of the needle assemblies when the given one of the needle assemblies is aligned with the first port.

In an exemplary embodiment, the needle assembly storage device further comprises a first cover and a second cover adapted to selectively cover the first and second ports. The first cover includes a leg adapted to engage a given one of the needle assemblies prior to the given one of the needle assemblies being aligned with the first port. Movement of the given one of the needle assemblies relative to the case causes the given one of the needle assemblies to push the leg and the first cover to uncover the first port. In an exemplary embodiment, the needle assembly storage device further comprises a first spring adapted to apply a biasing force on the first cover, and a second spring adapted to apply a biasing force on the second cover. In an exemplary embodiment, the needle assembly storage device further comprises a first resilient control arm coupled to the case. The first control arm includes a first hook adapted to releasably engage a first lug on the first cover and a first control element disposed adjacent the first port and adapted to engage the injection device. In an exemplary embodiment, the needle assembly storage device further comprises a second resilient control arm coupled to the case. The second control arm includes a second hook adapted to releasably engage a second lug on the second cover and a second control element disposed adjacent the second port and adapted to engage the injection device. When the injection device engages the first control element, the first control arm deflects, the first hook disengages the first lug and the first cover is displaced relative to the case under the biasing force of the first spring to cover the first port. When the injection device engages the second control element, the second control arm deflects, the second hook disengages the second lug and the second cover is displaced relative to the case under the biasing force of the second spring to cover the second port.

In an exemplary embodiment, a storage unit according to the present invention comprises a mounting sleeve having at least one resilient clip, a needle having a needle hub adapted to engage the at least one resilient clip, and at least one web detachably coupling the needle hub to the mounting sleeve. The needle hub includes a portion adapted to deflect the resilient clip when the needle moves in a distal direction relative to the mounting sleeve and abut the resilient clip when the needle moves in a proximal direction relative to the mounting sleeve. The storage unit includes an array of mounting sleeves in which consecutive mounting sleeves are coupled by a flexible connector.

Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1A: shows a top view of an unused needle assembly according to an exemplary embodiment of the present invention.
- Figure 1B: shows a perspective view of a storage unit for needle assemblies according to an exemplary embodiment of the present invention.
- Figure 1C: shows a sectional view of a storage unit according to an exemplary embodiment of the present invention.
- Figure 2A: shows a semitransparent top view of a needle assembly storage device according to an exemplary embodiment of the present invention.
- Figure 2B: shows a top view of a needle assembly storage device according to an exemplary embodiment of the present invention.
- Figure 3: shows a sectional view of an interface of a needle assembly storage device according to an exemplary embodiment of the present invention.
- Figure 4A-C: show a top view of a needle assembly storage device according to an exemplary embodiment of the present invention.
- Figure 5: shows a sectional view of an interface of a needle assembly storage device according to an exemplary embodiment of the present invention.
- Figure 6A-D: show a top view of a needle assembly storage device according to an exemplary embodiment of the present invention.

Corresponding parts are marked with the same reference symbols in all figures.

### Detailed Description

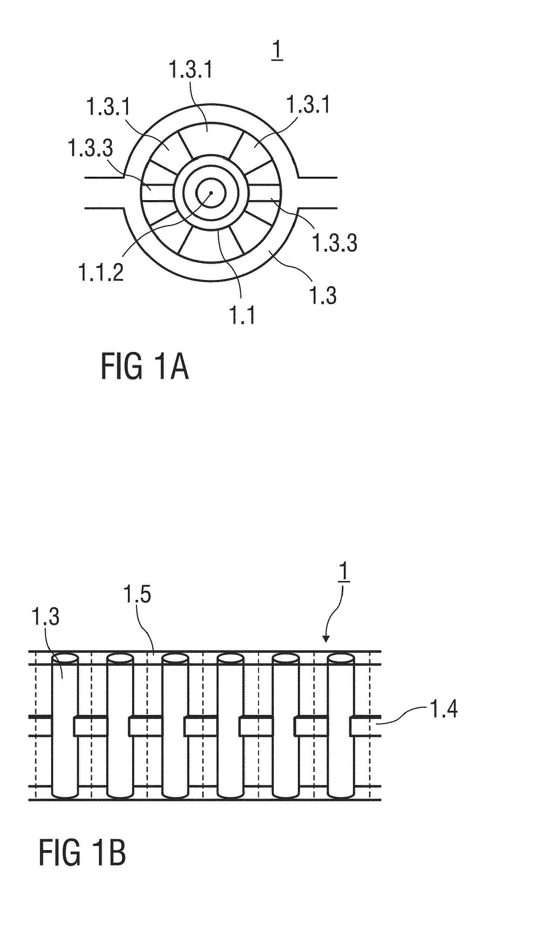

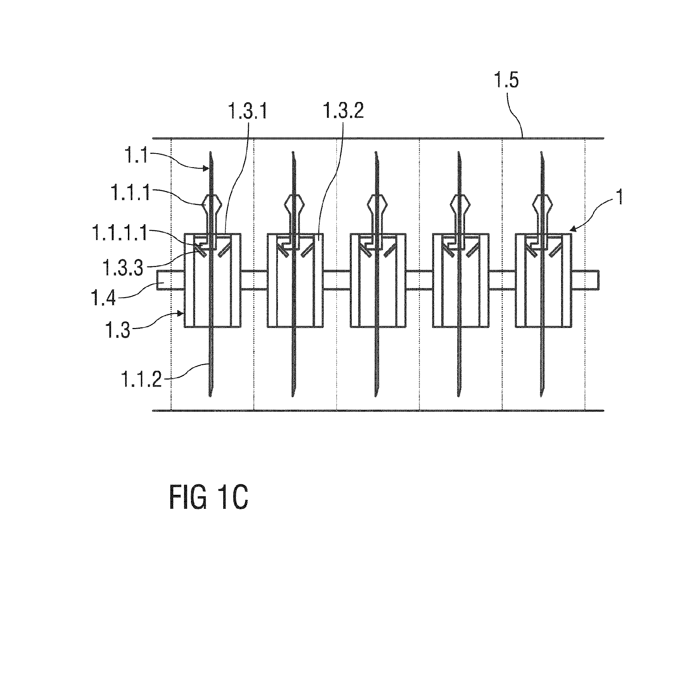

Figure 1A, Figures 1 B and 1C show a storage unit 1 for single-use needle assemblies according to an exemplary embodiment of the present invention.

As shown in Figure 1C, in an exemplary embodiment, the storage unit 1 is formed as an array of a plurality of unused needle assemblies 1.1. As explained further below, the storage unit 1 is also capable of containing used needle assemblies 1.1'. The needle assemblies 1.1, 1.1' may be individually mounted in mounting sleeves 1.3 and wrapped individually or collectively in a container 1.5, e.g., a blister pack or other package. The container 1.5 may be sealed to enclose all of the needle assemblies 1.1, 1.1' or sealed in a manner to enclose the needle assemblies 1.1, 1.1' individually.

In an exemplary embodiment, consecutive mounting sleeves 1.3 are coupled by a flexible connector 1.4, which may allow the consecutive mounting sleeves 1.3 to rotate relative to each other. The flexible connector 1.4 may also allow the storage unit 1 to be configured as a spiral or be folded.

In an exemplary embodiment, a needle assembly 1.1, 1.1' includes a needle 1.1.2 having a distal tip and proximal tip, and a needle hub 1.1.1 coupled to the needle 1.1.2. The needle hub 1.1.1 may include a proximal portion adapted to engage an adaptor on a medicament delivery device and a distal portion 1.1.1.1 adapted to engage resilient retaining clips 1.3.3 formed on the mounting sleeve 1.3. The distal portion 1.1.1.1 may be L-shaped, T-shaped, disc-shaped, or any other shape which includes a laterally extending hook or arm to engagement the retaining clips 1.3.3. In an exemplary embodiment, as shown in Figure 1C, the retaining clips 1.3.3 are hingedly coupled to the mounting sleeve 1.3, and in a non-deflected position, at least partially block a channel 1.3.2 in the mounting sleeve 1.3. Prior to use of the needle assembly 1.1, the distal portion 1.1.1.1 abuts the retaining clips 1.3.3, but a spring force in the hinge of the retaining clips 1.3.3 prevents the retaining clips 1.3.3 from deflecting. After use, when the needle assembly 1.1' is returned to the mounting sleeve 1.3, the needle assembly 1.1' is pushed distally relative to the mounting sleeve 1.3, causing the distal portion 1.1.1.1 to deflect the retaining clips 1.3.3 radially. After the distal portion 1.1.1.1 passes the retaining clips 1.3.3, the retaining clips 1.3.3 return to their non-deflected position and prevent retraction of the needle assembly 1.1' from the mounting sleeve 1.3 (by abutting the distal portion 1.1.1.1).

In an exemplary embodiment, the needle assembly 1.1 may be maintained in its pre-use position relative to the mounting sleeve 1.3 by one or more breakable webs 1.3.1, shown in Figure 1A, which are coupled to the needle hub 1.1.1 and the mounting sleeve 1.3. When the needle assembly 1.1 is engaged by the adapator on the medicament delivery device, pulling the delivery device in the proximal direction will cause the webs 1.3.1 to break and allow the needle assembly 1.1 to be withdrawn from the mounting sleeve 1.3.

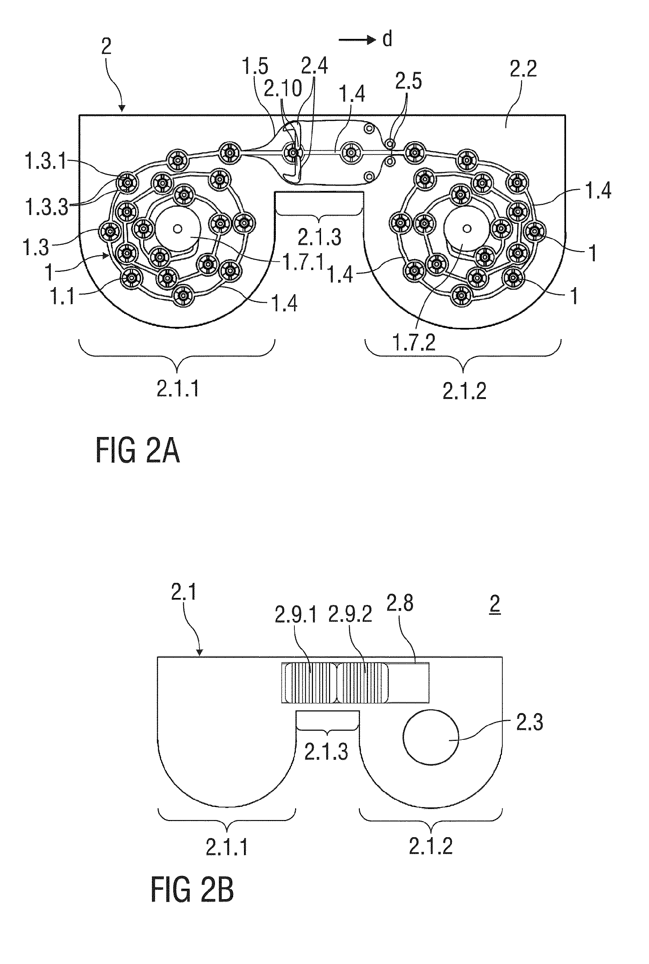

Figure 2A shows a section view of an exemplary embodiment of a needle assembly storage device 2 according to the present invention. The storage device 2 includes a case 2.1 defining a cavity. A first spool 1.7.1 is rotatably disposed in a first region 2.1.1 in the case 2.1 for unwinding the storage unit 1, and a second spool 1.7.2 is rotatably disposed in a second region 2.1.2 in the case 2.1 for winding the storage unit 1. In an exemplary embodiment, the spools 1.7.1, 1.7.2 may be spring-loaded or include brakes for ensuring that they rotate in only one angular direction. Thus, used needle assemblies 1.1' cannot be accessed.

In an exemplary embodiment, the case 2.1 may have a removable cover so that a used storage unit 1 can be replaced. In another exemplary embodiment, the case 2.1 may locked closed, preventing access to the cavity 2.3. In this exemplary embodiment, the storage device 2 may be disposable.

In an exemplary embodiment, the storage unit 1 may arranged as a spiral. To install the storage unit 1 in the case, a lagging end of the container 1.5 may be coupled to the first spool 1.7.1, and a leading end of the container 1.5 may fed over separators 2.4 to initiate separation of the container 1.5 and provide access to the unused needle assemblies 1.1. The leading end may then be fed through one or more rollers 2.5 and coupled to the second spool 1.7.2.

When the storage unit 1 is installed, the needle assemblies 1.1 can be advanced by actuation of an actuator 2.3, shown in Figure 2B. In an exemplary embodiment, the actuator 2.3 is a knob that is coupled to the second spool 1.7.2, such that rotation of the knob winds the storage unit 1 on the second spool 1.7.2 and unwinds the storage unit 1 from the first spool 1.7.1. In another exemplary embodiment, the actuator 2.3 may be a lever, a trigger, and/or any other manually-operated device and/or automatic device (e.g., motor) for advancing the storage unit 1. In an exemplary embodiment, the actuator 2.3 may include a brake or be spring-loaded to prevent actuation in a reverse direction. In an exemplary embodiment, the actuator 2.3 may include a stepping mechanism such that each actuation advances the storage unit 1 a step length for positioning the needle assemblies 1.1.

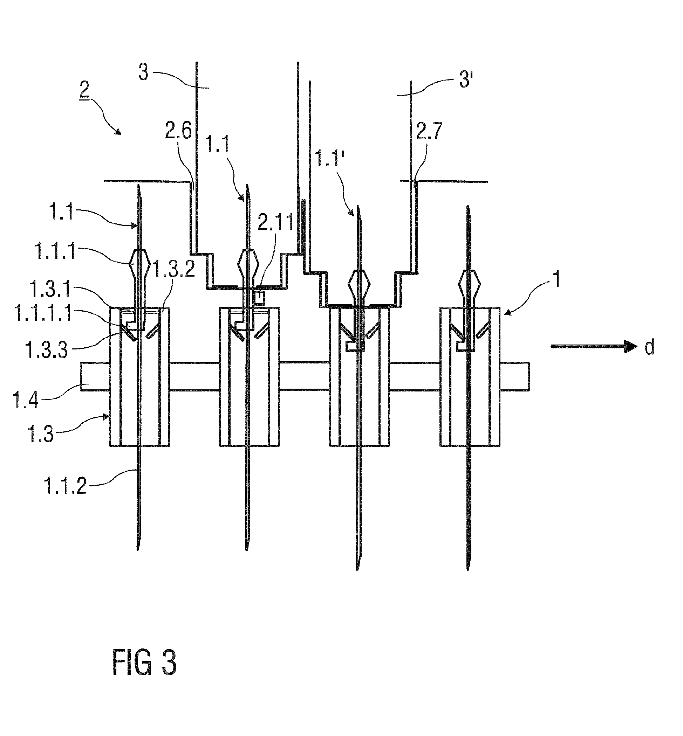

Figure 3 shows an exemplary embodiment of an interface 2.1.3 on the storage device 2 which provides access to the unused needle assemblies 1.1 and allows for return of the used needle assemblies 1.1'. The interface 2.1.3 includes a first port 2.6 adapted to provide access to the unused needle assemblies 1.1 and a second port 2.7 adapted to provide for the return of the used needle assemblies 1.1' to the storage unit 1. Generally, both ports 2.6, 2.7 are configured to receive an injection device 3. For example, the ports 2.6, 2.7 may have alignment features (e.g., abutments, geometric surfaces corresponding to shapes/sizes of the injection device 3) which properly orient the injection for engaging and disengaging a needle assembly.

When the injection device 3 is going to engage an unused needle assembly 1.1, the injection device 3 may be inserted into the first port 2.6 until it abuts the case 2.1. A resilient sleeve stopper 2.10 (shown in Figure 2A) formed in the case 2.1 adjacent the first port 2.6 may stop the next available mounting sleeve 1.3 containing an unused needle assembly 1.1 and align it with the first port 2.6, and a needle stopper 2.11 formed in the case 2.1 adjacent the first port 2.6 may stop the next available unused needle assembly 1.1 and align it with the first port 2.6. As in the injection device 3 is pressed into the first port 2.6, the resilient clips 1.3.3 and the webs 1.3.1 provide a force sufficient to resist any downward force applied to the unused needle assembly 1.1 by the injection device 3. Thus, the injection device 3 engages the unused needle assembly 1.1 (e.g., by engaging the needle hub 1.1.1), and when the injection device 3 is removed from the first port 2.6, the webs 1.3.1 break allowing the needle assembly 1.1. to be withdrawn from the mounting sleeve 1.3.

When the injection device is going to disengage a used needle assembly 1.1', the injection device 3 may be inserted into the second port 2.7 until it abuts the case 2.1. As in the injection device 3 is pressed into the second port 2.7, the resilient clips 1.3.3 deflect, until the distal portion 1.1.1.1 of the needle hub 1.1.1 bypasses the resilient clips 1.3.3. Then, the resilient clips 1.3.3 return to their non-deflected position and engage the distal portion 1.1.1.1 of the needle hub 1.1.1 when the injection device 3' is withdrawn from the second port 2.7, separating the used needle assembly 1.1' from the injection device 3.

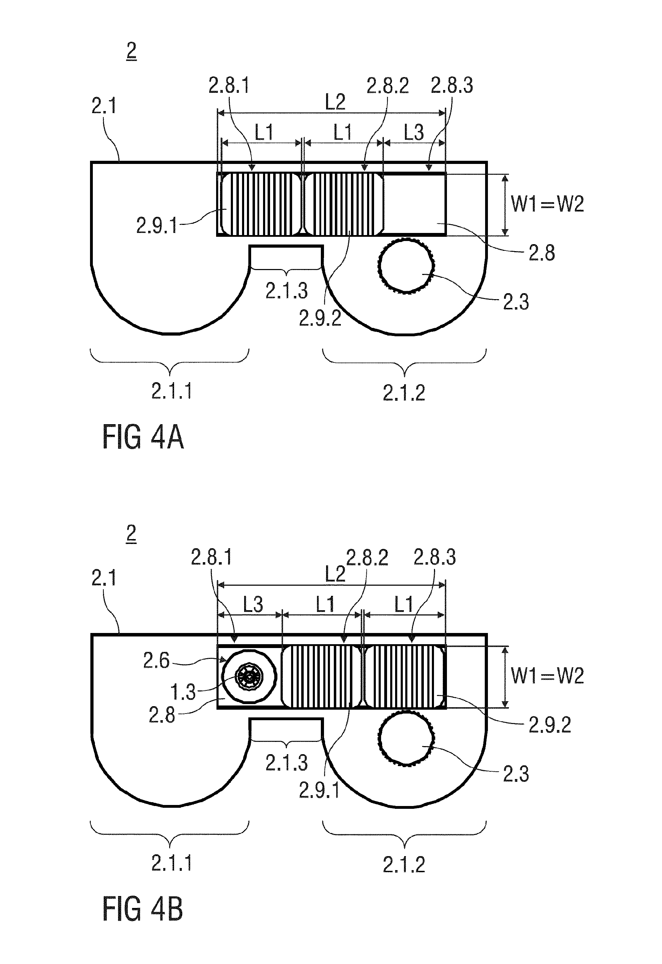

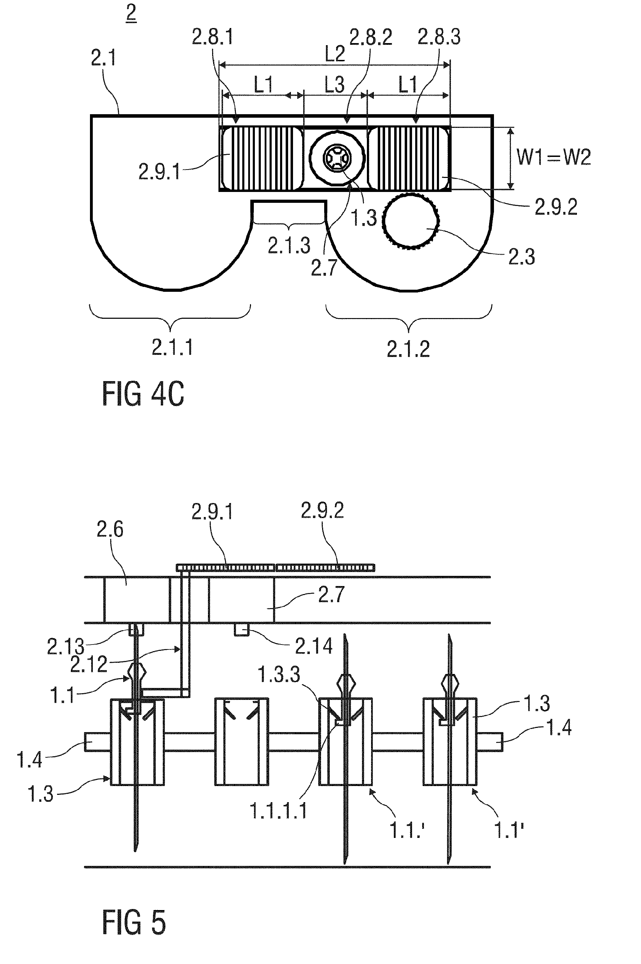

Figures 4A, 4B and 4C show an exemplary embodiment of a cover mechanism. In an exemplary embodiment, the cover mechanism includes two covers 2.9.1, 2.9.2 slidably disposed in a channel 2.8 on the case 2.1. The covers 2.9.1, 2.9.2 are adapted to selectively cover the first and second ports 2.6, 2.7. The covers 2.9.1, 2.9.2 may have a length L1, and the channel 2.8 may have a length L2 which is three times L1, to allow for movement of the covers 2.9.1, 2.9.2 in the channel 2.8. In another exemplary embodiment, the total length L2 of the channel 2.8 may be two times L1 plus an additional length L3 which is shorter than L1 but greater than a diameter of the ports.

As shown in Figure 4A, the first cover 2.9.1 is in a first section 2.8.1 in the channel 2.8 covering the first port 2.6, the second cover 2.9.2 is in a second section 2.8.2 in the channel 2.8 covering the second port 2.7, and a third section 2.8.3 in the channel 2.8 is unoccupied. The third section 2.8.3 may have a length L3 which is less than the length L1. The length L3 may correspond to a diameter of the respective ports 2.6, 2.7.

When access to an unused needle assembly 1.1 is desired, the first and the second covers 2.9.1, 2.9.2 may be moved toward the third section 2.8.3, as shown in Figure 4B, to uncover the first port 2.6. When access to the second port 2.7 is desired for discarding a used needle assembly 1.1', the first cover 2.9.1 may be returned to the first section 2.8.1 and the second cover 2.9.2 may be moved to the third section 2.8.3, as shown in Figure 4C.

In an exemplary embodiment, the actuator 2.3 may be operably coupled to the covers 2.9.1, 2.9.2. For example, sliding the covers 2.9.1, 2.9.2 to reveal the first port 2.6 may advance the storage unit 1 one step to the next available unused needle assembly 1.1. and sliding the covers 2.9.1, 2.9.2 to reveal the second port 2.7 may advance the storage unit 1 one step to reveal the next available empty mounting sleeve 1.3.

Figure 5 shows an exemplary embodiment of a mechanism for moving the covers 2.9.1, 2.9.2 in conjunction with movement of the storage unit 1. A leg 2.12 is coupled to the first cover 2.9.1 and is adapted to abut the needle hub 1.1 and/or the mounting sleeve 1.3 for an unused needle assembly 1.1 went it is adjacent to the first port 2.6. As the storage unit 1 is advanced, and the mounting sleeve 1.3 moves into alignment with the first port 2.6, the leg 2.12 is pushed in the same direction as the advancing storage unit 1, thereby displacing the first cover 2.9.1 to reveal the first port 2.6, as shown in Figure 5.

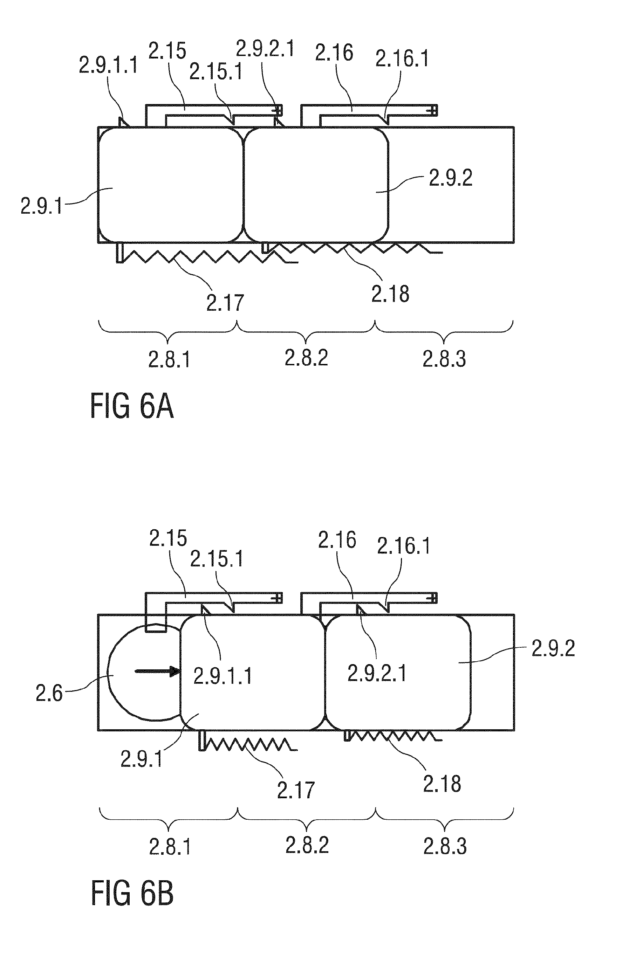

Figures 6A through 6D show an exemplary embodiment of a release mechanism for the covers 2.9.1, 2.9.2. Figure 6A shows the covers 2.9.1, 2.9.2 covering the respective first and second ports 2.6, 2.7. In this exemplary embodiment, the first cover 2.9.1 includes a first lug 2.9.1.1 adapted to engage a first hook 2.15.1 on a first resilient control arm 2.15 disposed adjacent the channel 2.8, and the second cover 2.9.2 includes a second lug 2.9.2.1 adapted to engage a second hook 2.16.1 on a second resilient control arm 2.16 disposed adjacent the channel 2.8. The first cover 2.9.1 is coupled to a first spring 2.17, and the second cover is coupled to a second spring 2.18.

As shown in Figure 6B, when the storage unit 1 is advanced, the needle hub 1.1 and/or the mounting sleeve 1.3 for the next unused needle assembly 1.1 abuts and pushes the leg 2.12 which concurrently displaces the covers 2.9.1, 2.9.2 to reveal the first port 2.6. As the covers 2.9.1, 2.9.2 are displaced, the springs 2.17, 2.18 are compressed.

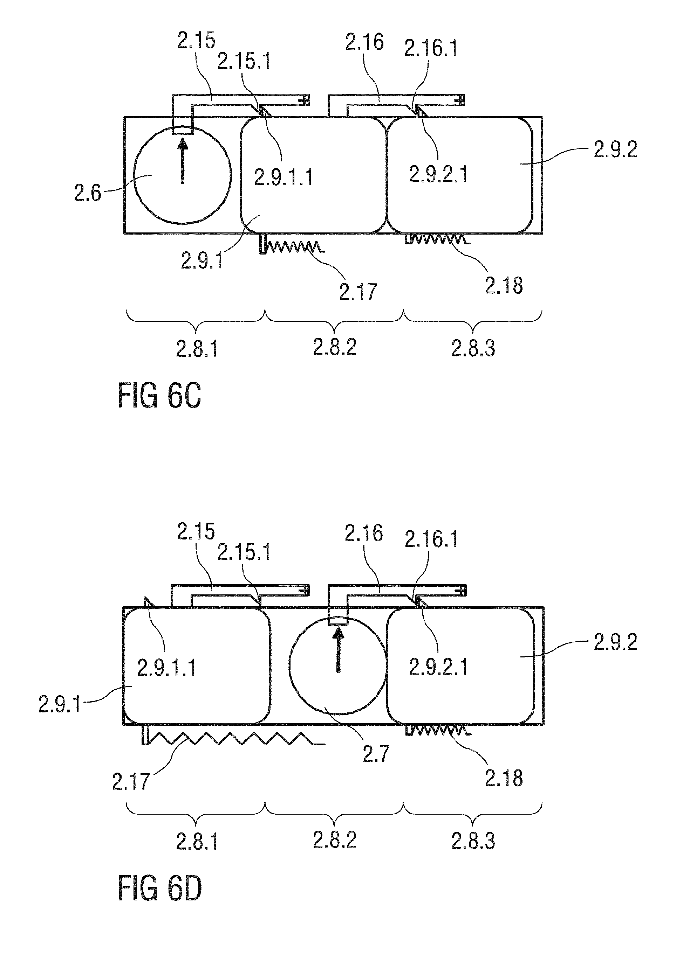

As shown in Figure 6C, when the covers 2.9.1, 2.9.2 have been displaced to fully reveal the first port 2.6, the lugs 2.9.1.1, 2.9.2.1 have engaged the hooks 2.15.1, 2.16.1 on the control arms 2.15, 2.16. The engagement of the lugs 2.9.1.1, 2.9.2.1 and the hooks 2.15.1, 2.16.1 prevents the covers 2.9.1, 2.9.2 from moving under the force of the springs 2.17, 2.18.

When an injection device 3 is inserted into the first port 2.6 to engage an unused needle assembly 1.1, the injection device 3 may engage a first control element 2.13 (shown in Figure 5) which is coupled to the first control arm 2.15. The first control element 2.13 may be a projection which resides in a space formed in the first port 2.6. When the injection device 3 engages the control element 2.13, the first control element 2.13 is displaced within the space. Displacement of the first control element 2.13 causes the first control arm 2.15 to deflect and disengage the first hook 2.15.1 from the first lug 2.9.1.1, allowing the first cover 2.9.1 to move within the channel 2.8 under the force of the first spring 2.17 back toward the first section 2.8.1.

As shown in Figure 6D, movement of the first cover 2.9.1 uncovers the second port 2.7, and removal of the injection device 3 from the first port 2.6 allows the first control arm 2.15 to return to its non-deflected position. When an injection device 3' is inserted into the second port 2.7 to disengage a used needle assembly 1.1', the injection device 3 may engage a second control element 2.14 (shown in Figure 5) which is coupled to the second control arm 2.16. The second control element 2.14 may be a projection which resides in a space formed in the second port 2.7. When the injection device 3 engages the second control element 2.14, the second control element 2.14 is displaced within the space. Displacement of the second control element 2.14 causes the second control arm 2.16 to deflect and disengage the second hook 2.16.1 from the second lug 2.9.2.1, allowing the second cover 2.9.2 to move within the channel 2.8 under the force of the second spring 2.18 back toward the second section 2.8.2. Removal of the injection device 3 from the second port 2.6 allows the second control arm 2.16 to return to its non-deflected position.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. A needle assembly storage device (2) comprising:
a case (2.1) having a first port (2.6) and a second port (2.7);
a first spool (1.7.1) rotatably disposed in the case (2.1) and adapted to support a storage unit (1) of needle assemblies;
a second spool (1.7.2) rotatably disposed in the case (2.1) and adapted to support the storage unit (1) of the needle assemblies; and
an actuator (2.3) adapted to rotate the first spool (1.7.1) or the second spool (1.7.2) to advance the storage unit (1) past the first port (2.6) and the second port (2.7).

2. The needle assembly storage device (2) according to claim 1, further comprising:
a separator (2.4) disposed in the case (2.1) adjacent the first port (2.6), the separator (2.4) adapted to separate a container (1.5) surrounding the needle assemblies.

3. The needle assembly storage device (2) according to claim 2, further comprising:
at least one roller (2.5) disposed in the case (2.1) adjacent the second port (2.7), the at least one roller (2.5) adapted to re-wrap the needle assemblies in the container (1.5).

4. The needle assembly storage device (2) according to any one of the preceding claims, further comprising:
a sleeve stopper (2.10) disposed in the case (2.1) adjacent the first port (2.6), the sleeve stopper (2.10) adapted to releasably engage a given one of the needle assemblies when the given one of the needle assemblies is aligned with the first port (2.6).

5. The needle assembly storage device (2) according to any one of the preceding claims, further comprising:
a needle stopper (2.11) disposed in the case (2.1) adjacent the first port (2.6), the needle stopper (2.11) adapted to engage a given one of the needle assemblies when the given one of the needle assemblies is aligned with the first port (2.6).

6. The needle assembly storage device (2) according to any one of the preceding claims, further comprising:
a first cover (2.9.1) and a second cover (2.9.2) adapted to selectively cover the first and second ports (2.6, 2.7).

7. The needle assembly storage device (2) according to claim 6, wherein the first cover (2.9.1) includes a leg (2.12) adapted to engage a given one of the needle assemblies prior to the given one of the needle assemblies being aligned with the first port (2.6).

8. The needle assembly storage device (2) according to claim 7, wherein movement of the given one of the needle assemblies relative to the case (2.1) causes the given one of the needle assemblies to push the leg (2.12) and the first cover (2.9.1) to uncover the first port (2.6).

9. The needle assembly storage device (2) according to claim 6, further comprising:
a first spring (2.17) adapted to apply a biasing force on the first cover (2.9.1); and
a second spring (2.18) adapted to apply a biasing force on the second cover (2.9.2).

10. The needle assembly storage device (2) according to claim 6, further comprising:
a first resilient control arm (2.15) coupled to the case (2.1), the first control arm (2.15) including a first hook (2.15.1) adapted to releasably engage a first lug (2.9.1.1) on the first cover (2.9.1) and a first control element (2.13) disposed adjacent the first port (2.6) and adapted to engage the injection device (3); and
a second resilient control arm (2.16) coupled to the case (2.1), the second control arm (2.16) including a second hook (2.16.1) adapted to releasably engage a second lug (2.9.2.1) on the second cover (2.9.2) and a second control element (2.14) disposed adjacent the second port (2.7) and adapted to engage the injection device (3).

11. The needle assembly storage device (2) according to claims 9 and 10, wherein, when the injection device (3) engages the first control element (2.13), the first control arm (2.15) deflects, the first hook (2.15.1) disengages the first lug (2.9.1.1) and the first cover (2.9.1) is displaced relative to the case (2.1) under the biasing force of the first spring (2.17) to cover the first port (2.6).

12. The needle assembly storage device (2) according to claims 9 and 10, wherein, when the injection device (3) engages the second control element (2.14), the second control arm (2.16) deflects, the second hook (2.16.1) disengages the second lug (2.9.2.1) and the second cover (2.9.2) is displaced relative to the case (2.1) under the biasing force of the second spring (2.18) to cover the second port (2.7).

13. A storage unit (1) for needle assemblies for use with the needle assembly storage device (2) according to any of the preceding claims, the storage unit (1) comprising:
a mounting sleeve (1.3) having at least one resilient clip (1.3.3);
a needle (1.1.2) having a needle hub (1.1) adapted to engage the at least one resilient clip (1.3.3); and
at least one web (1.3.1) detachably coupling the needle hub (1.1) to the mounting sleeve (1.3).

14. The storage unit (1) according to claim 13, wherein the needle hub (1.1) includes a portion (1.1.1.1) adapted to deflect the resilient clip (1.3.3) when the needle (1.1.2) moves in a distal direction relative to the mounting sleeve (1.3) and abut the resilient clip (1.3.3) when the needle (1.1.2) moves in a proximal direction relative to the mounting sleeve (1.3).

15. The storage unit (1) according to claim 13, wherein the storage unit (1) includes an array of mounting sleeves (1.3), wherein consecutive mounting sleeves (1.3) are coupled by a flexible connector (1.4).
